# EUROPEAN PATENT SPECIFICATION

(11) **EP 1 007 629 B1**
(45) Date of publication and mention of the grant of the patent: **24.09.2003**
(21) Application number: 98904261.9
(22) Date of filing: 20.02.1998
(51) Int. Cl.: C12N 5/00

(54) **SUGAR CANE PRODUCTION**
HERSTELLUNG VON ZUCKERROHR
PRODUCTION DE CANNE A SUCRE

(30) Priority: 21.02.1997 GB 9703628
(43) Date of publication of application: 14.06.2000
(73) Proprietor: Abdelrahman, Layla Zakaria, Didsbury, Manchester M20 6JZ (GB)
(72) Inventor: Abdelrahman, Layla Zakaria, Didsbury, Manchester M20 6JZ (GB)
(74) Representative: Quest, Barry
(86) International application number: GB9800387
(87) International publication number: WO98037173

(56) References cited:
- EP-A- 0 608 716
- FR-A- 2 570 959
- US-A- 4 586 288
- US-A- 5 254 802
- US-A- 5 409 828
- US-A- 5 623 781
- VASIL, I.K.: "Advantages of embryogenic cell cultures of graminae" IAEA-SM, vol. 282, no. 41, 1986, pages 71-75, XP002077010
- KRESOVICH, S. ET AL.: "Comparison of the in vitro responsiveness of callus cultures derived from immature inflorescence and leaf base tissues of two interspecific hybrids of sugar cane" SUGAR CANE, no. 6, 1985, pages 8-10, XP002077011
- PATENT ABSTRACTS OF JAPAN vol. 095, no. 006, 31 July 1995 & JP 07 079656 A (GUMMA PREF GOV), 28 March 1995
- PATENT ABSTRACTS OF JAPAN vol. 018, no. 030 (C-1153), 17 January 1994 & JP 05 260870 A (HOKKO CHEM IND CO LTD), 12 October 1993
- PATENT ABSTRACTS OF JAPAN vol. 098, no. 002, 30 January 1998 & JP 09 266788 A (SNOW BRAND MILK PROD CO LTD), 14 October 1997
- PATENT ABSTRACTS OF JAPAN vol. 014, no. 290 (C-0731), 22 June 1990 & JP 02 092220 A (JAPAN TOBACCO INC), 3 April 1990
- PATENT ABSTRACTS OF JAPAN vol. 018, no. 490 (C-1249), 13 September 1994 & JP 06 165624 A (IWATE PREF GOV), 14 June 1994
- PATENT ABSTRACTS OF JAPAN vol. 014, no. 208 (C-0714), 27 April 1990 & JP 02 046240 A (NITSUSEN KAGAKU KOGYO KK), 15 February 1990
- LIU, M.C.: "In Vitro methods applied to sugar cane improvement" PLANT TISSUE CULTURE,1981, pages 299-323, XP002077012 TAIWAN

## Description

This invention relates to sugar cane plants and more particularly to methods of producing such plants.

Sugar cane is well known as an extremely important crop plant because of its use in the production of foodstuffs and the use of its by-products such as molasses, bagasse, filter mud and ethanol, all of which are of great value to both developing and developed countries.

Generally, sugar cane is cultivated as a perennial crop. The plant is allowed to grow for one year and is known as "plant cane". The plant cane is cut at this stage and processed to produce sugar. The plant cane root and a small amount of cane stem is left in the ground and this grows over the next year to produce the "first ratoon". This is then harvested to produce sugar and the residue of the plant is left to grow for a further year to produce the "second ratoon". In some countries this growing cycle is repeated up to a "fifth ratoon" before the plant is finally uprooted.

Sugar cane is wind-pollinated and this leads to great variability in the plants obtained from seeds. This is useful for breeding programmes because it produces many different plant variations which can be used to select new useful clones. However, for normal commercial propagation the natural reproductive method does not give true-to-type plants. As a result, up until now, vegetative propagation has been the only practical means of propagating sugar cane. The existing method of propagating sugar cane relies on using the stems of mature sugar cane. This method is very wasteful in terms of reducing the overall productivity of the sugar cane crop, is expensive in terms of labour costs and can also produce problems in terms of spreading virus diseases such as Fiji disease, bacterial diseases such as red rot and the main fungal disease of smut. Disease problems in sugar cane production can be extremely serious since they are spread by many different vectors and can very rapidly spread throughout sugar cane plantations.

Unfortunately, vegetative propagation is very expensive because the plant has to grow for 9-12 months before the stem can be cut down into segments of about 30 cm. The main problem is that the cut ends of the cane are exposed to contamination, especially due to the presence of the nutrient sucrose in the cane sap. As a result, it is easy to spread viral, bacterial and fungal diseases which can have serious effects on sugar cane production.

Because of all these problems with the production of new sugar cane plants, tissue culture methods have been developed for breeding and propagation programmes and for the elimination of viruses. For example, Barredo R., Luzaman R and Dequinto B. (1994) used the spindle leaf (the innermost leaf of the tiller) to produce 13,500 plantlets from variety Phil 74-64. However, they found that it took 8 months from the beginning of the laboratory work to produce seedlings capable of being planted in the field. Barba R.C., Zamora A.B., Linga C.K. and Thai Van N. (1975) used a callusing method which allowed them to produce 4,000 plantlets from a 3 cm piece of sugar cane shoot but disadvantageously this method can lead to genetic variations.

Recent advances in biotechnology have offered new opportunities for crop improvement and propagation through plant tissue culture techniques. A method according to the preamble of claim 1 is known from Vasil I, K, ("Advantages of embryogenic cell cultures of graminae", IAEA-SM, Vol. 282, no. 41, 1986, pp. 71-75).

Somatic embryos are bipolar structures which contain both shoot and root meristems. They may be formed in both callus and cell suspension culture and lead directly to the formation of mature plants. Somatic embryogenesis offers a potential system for large scale production of plants. However, for somatic embryogenesis to be practical for the large scale production of plants, numerous problems have to be overcome which, up until the present invention, have remained obstacles to the use of the method for the production of sugar cane plants. Up until the present invention, mature somatic embryos have only been produced from callus grown on solid medium. Cells grown in callus are known to be genetically unstable and so the embryos grown from callus are likely to be genetically distinct. Moreover, callus-derived embryos do not grow in synchrony and manipulation is labour-intensive. Such problems render callus-derived somatic embryogenesis unsuitable for large-scale commercial propagation. By contrast, cell suspensions in liquid culture show greater genetic stability, can be synchronised by modifying growth conditions and can be subjected to automated manipulation. Up until the present invention, attempts at liquid culture only succeeded in producing immature globular embryos.

The present invention has been provided through a consideration of the above mentioned problems. The present invention addresses those problems and provides an efficient and reliable method of producing sugar cane plants which is particularly suitable for use on a large scale for commercial purposes.

According to the present invention there is provided a method of producing sugar cane somatic embryos from sugar cane explants comprising the steps of:
(1) culturing immature embryos from explants
(2) culturing mature sugar cane somatic embryos from those immature embryos, whereby at least step (2) occurs in liquid suspension culture.

The present invention provides a system involving somatic embryogenesis for the production of somatic embryos for use in the production of sugar cane plant as mature somatic embryos may be produced quickly and in large quantities and the original plant gene characteristics are maintained in the somatic embryos.

The sugar cane somatic embryos produced by the method of the present invention may subsequently be germinated to produce sugar cane plants or may be encapsulated in an encapsulating agent to produce "artificial seeds" for direct delivery to the field for subsequent germination of the embryos *in situ.*

The production of sugar cane somatic embryos by the method of the present invention involves somatic embryogenesis. This method is extremely valuable because it is possible to produce vast numbers of embryos in small volumes of culture media in an approximately synchronous manner. A high multiplication rate, however, is only the first of several potential advantages offered by somatic embryogenesis for the subsequent production of sugar cane plants in comparison with other methods of vegetative propagation of sugar cane plants. For example, both the growth of embryogenic tissue in step (1) and the subsequent development of the embryos to maturity in step (2) can be accomplished in liquid medium, making possible the manipulation of very large numbers of propagules with minimal handling. In addition, the product of somatic embryogenesis is an embryo that is capable of developing into a regenerated sugar cane plant with very little further input of labour. The advantage of somatic embryogenesis for the production of sugar cane plants over the prior art systems of vegetative propagation and micropropagation is the presence of both root and shoot meristems in the same unit and therefore there is no requirement for the laborious transfer operations, thus reducing operating costs very significantly.

In addition, this absence of repetitive transfer operations advantageously decreases contamination which is spread by contact. A further advantage is that the embryogenic systems are capable of producing separated individual embryos, unattached to either mother tissue or other embryos. Thus, embryogenic cultures produce propagules that are not only complete but also discrete. The combination of those two properties gives somatic embryos potential for direct delivery to the greenhouse or field for example, as components of artificial seeds or in a fluid drilling system.

The method of the present invention advantageously provides an in *vitro* multiplication tool providing the new possibility of quickly obtaining large quantities of sugar cane embryos in a liquid medium. This permits much greater and faster propagation of sugar cane plants than can presently be obtained using other techniques, such as micropropagation, which suffer from high labour costs.

Sugar cane plants, regenerated using the method of the present invention which involves somatic embryogenesis, are most preferably derived from characteristic organised meristems or meristematic cells of sugar cane plants. These cells are by nature genetically stable and not prone to mutational changes. Indeed, there is evidence that there is strong selection in favour of genetically normal cells during somatic embryo development. Consequently, sugar cane plants derived from the method of the present invention give rise to truly clonal populations which is important for the reliable and efficient mass production of sugar cane plants.

Embryogenic suspension cultures are particularly suitable for large scale production because of the absence of repetitive handling of the plant tissue, the potential to easily scale-up the process and the relative ease of automating the process. The development of the process into a practical production system requires, ideally, that single embryos are produced, that the embryos are at the same stage of development and that there is ultimately a high conversion rate into seedlings. The advantage of using embryos as propagules is that it is possible to encapsulate them for direct delivery to the field.

Furthermore the method of the present invention advantageously provides means by which the sugar cane plants can be regenerated from cells which have been selected in manipulated cultures. Accordingly, the method of the present invention provides an efficient method for use in sugar cane novel genetic manipulation techniques for crop improvement, such as somatic hybridization and genetic transformation.

The explant used in step 1 of the method of the present invention may be taken from any part of and at any stage of development of a sugar cane mother plant. For example, the source of explant can be from the leaf, root, young shoot, buds, infloresence, young internodes of the sugar cane plant and/or the explants may comprise mature embryos derived by stressing leaf, young shoot, root, buds, infloresence or young internodes of the sugar cane plant. Stressing may be achieved by treating the plant part with 95% ethanol for a period of time (preferably 1 - 5 hours) and/or cooling the plant part to a temperature of approximately 5 - 15°C for approximately 1 - 3 months. Most preferably root-derived or mature embryos are used as the explant material.

Root explant is advantageously most productive in forming immature embryos, which is probably due to the root (particularly the tip) consisting mainly of meristematic tissues. Furthermore, root explant advantageously is less affected by the known problem of "browning" of culture media and cells in plant tissue culture techniques. The "browning" effect is produced by the oxidation of phenolic compounds to quinone oxidation products. Phenolic derivatives are often released by explants. Since browning of the immature embryo culture when derived from root explants is substantially absent, advantageously there is no need to change the culture medium and accordingly, root-derived cultures tend to accumulate more bio-mass over any fixed period of time.

A further advantage of using root-explant rather than leaf or shoot explants is that a fine, highly dispersed immature embryo culture is generated. Moreover, root explants may be used to generate embryos in liquid suspension culture directly, without the need for the formation of intermediate callus and without the need to change the culture media every 2 to 5 days. This feature is very important for the application of the method of the present invention industrially as a means of propagation of sugar cane using somatic embryogenesis. Culture of root explant together with non-root explant confers substantially the same advantages on the mixed culture as those above described advantages for root explant culture alone. For example, where "browning" would normally occur in shoot-explant culture, such browning is minimized in a mixed shoot and root explant culture.

Furthermore, root explants are readily available since roots are easily induced by placing shoot material, including at least one node, in distilled water. After a period of time, a root network grows, from which explant material may be sourced. This is particularly useful in the industrial application of the method of the present invention.

The advantages of using leaf explant include their ready availability and that they may usually be sourced in an uncontaminated state.

Where leaf explants are used, most preferably the leaf explant is sourced from an area close to the growing point of the plant since the frequency of browning of the culture medium increases with increasing distance from the growing point. Furthermore, the age of the donor plant from which the leaf explant is sourced is preferably between approximately 3-12 months, but most preferably approximately 9 months as the frequency of browning of the culture media is substantially absent and virtually all leaf segments from a 9 month old plant are highly embryogenic.

The above mentioned advantages of using root explant, alone or in combination with other sources of explant, and in particular for the avoidance of the frequently encountered problems of "browning" in plant culture techniques, are beneficial for culture of all types of plants (such as trees, date palm, potatoes) and accordingly use of sugar cane root explant is not limited to the culture of sugar cane.

The method of culturing immature embryos from explants (step 1 of the present invention) preferably comprises the steps of:
(a) culturing explant material on culture medium to produce somatic cells
(b) selecting and multiplying somatic cells derived from the explant material
(c) culturing the somatic cells for a period of time on culture medium to induce immature sugar cane embryos from the somatic cells

The immature embryos may be initiated and maintained as embryogenic callus on solid media or as an embryogenic cell suspension culture in liquid media or on a combination of both solid and liquid media preferably as a "double layer" medium.

A wide range of solid and/or liquid culture media may be used in the method of the present invention from relatively dilute media to the more concentrated formulations of Evans et al, Gamborg et al, Schenk and Hildebrandt (SH medium) and Murashige and Skoog (MS medium). Most preferably solid or liquid MS medium is used for the method of the present invention as it is particularly advantageous for formation of immature embryos as embryogenic callus/suspension culture and for the induction of somatic embryogenesis thereof possibly due to the presence of higher levels of ammonia nitrogen in MS medium. Different media may be used in the different steps of the method of the present invention as required.

Most preferably plant growth regulators such as auxins (eg 2,4 - dichlorophenoxyacetic acid (2,4-D), naphthaleneacetic acid (NAA), indole 3-acetic acid (IAA)), cytokinins, gibberellins, abscissic acid, anti-auxins, kinetin, zeatin and/or activated charcoal are included either alone or in combination in the media formulation. 2,4-D auxin is the most effective regulator for the induction of immature embryo (callus formation and/or suspension culture) development from explants and for the development of somatic embryos from the immature embryos in the method of the present invention. A wide range of concentrations of 2,4-D are suitable, for example 0.5-10mg/l, but most preferably a relatively high concentration is used such as approximately 3-5 mg/l. In contrast, cytokinins have an inhibitory effect on the growth of immature sugar cane embryos. Preferably ABA is included together with 2,4-D in the culture media, at a concentration of 0.1-20 mg/l and most preferably at a concentration of approximately 1mg/l. ABA advantageously retards the growth of non-embryogenic cells so that the percentage of embryogenic cells in culture is optimized.

As carbon and energy sources, carbohydrates are a major and essential constituent of a tissue culture medium. Most preferably sucrose is used as the major carbohydrate constituent of the media used in the method of the present invention for the culture of sugar cane. Many other monosaccharide, such as glucose and fructose, disaccharides such as lactose and other sugars such as melibiose can also support growth and embryogenesis in the culture method of the present invention. However, the use of sucrose is particularly advantageous for the method of the present invention as it does not increase culture browning. The concentration of sucrose used is preferably in the range 10-60g/l. Most preferably the concentration of sucrose used is approximately 30g/l. Up until the present invention it has been usual in the culture of sugar cane for coconut water to be a constituent of the culture medium. However, advantageously in the method of the present invention coconut water is not a required constituent, thus reducing costs.

The production of embryogenic callus and/or embryogenic suspension culture is most effectively stimulated by stressing the explant material and/or the tissue from which the explant material is sourced prior to introduction of the explant material to the medium (step a).

For example, this be may achieved by a stress-treatment involving cooling the explants to a temperature of approximately 5°C - 15°C for approximately 1-3 months. Most preferably the explants are cooled to approximately 10°C for approximately 2 months. Other suitable stress-treatments to stimulate embryo production include soaking the explants in approximately 95% ethanol for approximately 1-5 hours. The latter ethanol method is preferred as this is a ready method of treating sugar cane explants to remove contamination whilst leaving the cells viable for culture to form embryonic material. The most preferable time period for soaking the explants in ethanol is approximately 4 hours.

Preferably the exposed cut ends of tissue donated from the mother sugar cane plant are coated with wax, such as candle wax, prior to ethanol treatment to reduce the absorption of ethanol by the tissue exposed at the cuts. After soaking the tissue in ethanol preferably the tissue is wrapped in sterilized dry tissue paper or the like to absorb the surplus ethanol. Explant material may then be selected and dissected from the donor tissue (eg leaf, shoot, root) and introduced to the culture medium. The explant is then cultured for a period of time to induce the formation of somatic cells. Somatic cell development is most preferably positively selected by using a culture medium which comprises ABA. ABA retards the growth of other types of cells so optimizing the percentage of the desired somatic cells. Somatic cells are further selected by a method which separates cells according to their size and shape. Sugar cane somatic cells are typically rounded in shape having a diameter of approximately 40 - 65 µm and most typically between 46 - 63 µm. In contrast non-embryogenic sugar cane cells are typically elongate and less than approximately 46 µm in length. A convenient method of selecting substantially only somatic cells is to sieve the culture through a first sieve of 100 - 50 µm mesh size, which thus substantially allows somatic and non embryogenic cells to pass through (but collects any larger particles such as cell aggregates), and then subsequently to sieve that sieved culture through a second sieve having a mesh size of 50 - 38 µm and which substantially allows non-embryogenic cells to pass through whilst substantially collecting the somatic cells. Optimal selection of somatic cells occurs where the first sieve is around 63 µm and the second sieve is around 45 µm.

The selected somatic cells are then preferably resuspended in the above described 2,4D containing media, which preferably also contains ABA as above described, for multiplication purposes.

The somatic cells are then cultured for a period of time to form immature embryos, step (c). Generally the time period is between 10 and 40 days depending upon, for example, the nature of the explant, culture media used and concentration and type(s) of growth regulators used. Generally, the percentage of somatic cells forming immature embryos increases with the concentration of 2,4-D.

Immature embryos may be produced by the method of the present invention in dark or light conditions or a combination of both dark and light conditions. Most preferably the culture is exposed to photo period cycles of 16 hours of light and 8 hours of dark as this cycle produces the greatest number of embryos.

The culturing of immature embryos from somatic cells derived from explants may involve growth of sub-cultures from the initial culture. Most preferably initial culture is maintained for at least three months on the initial medium before subculturing to fresh media. This has been found to produce the optimal number of embryogenic cultures.

Where the initial culture of explant involves the initiation of embryogenic callus from explants on solid media, step 1 of the method of the present invention further involves the subculturing of embryogenic callus from the explant in liquid media to establish and maintain embryogenic cell suspension cultures. Up until the present invention, such sub-culturing presented numerous problems which include extensive browning of the callus cultures when transferred to liquid medium, the production of a large number of roots rather than embryos in liquid culture, the aggregation of the callus cells rather than formation of a fine suspension culture etc.

Previously, the browning of the liquid culture medium was only prevented by repeatedly changing the culture medium every 3-5 days. Such changes are time consuming and can waste expensive medium. The medium changes can also introduce the possibility of culture contamination and loss of important chemicals produced by cells. Medium replacement to overcome browning problems is therefore not a practicable option for the large scale propagation of sugar cane via somatic embryogenesis. A different solution to this problem has been found by the present invention which does not require such repeated sub-culturing for the avoidance of browning.

In the present invention most preferably embryonic callus formed from root explant either alone or in combination with other types of explant is used to form the liquid suspension culture. Root callus disperses quickly and produces a fine suspension with minimal cell aggregates. Furthermore root callus substantially does not produce any medium or cell browning and thus suspension cultures of root callus do not require further sub culturing to avoid the problems of medium and/or cell browning. The method of the present invention using sugar cane root explant for culture is important for all types of plant culture which, up until the present invention have suffered from "browning" problems such as date palm.

At least in the case of leaf explant derived cultures ascorbic acid and citric acid assist in preventing browning of cells and culture media. Ascorbic acid is accordingly preferably included as a constituent of the callus initiation solid medium and the liquid embryogenic suspension medium. Different concentrations of ascorbic acid /citric acid are required at different stages of the culture process of the present invention. Most preferably in the initiation of callus from leaf explant approximately 1-2mg/l of ascorbic acid is included in the solid media to prevent explant browning. For the initiation of suspension culture from callus, preferably 50 mg/l to 200mg/l of ascorbic acid and most preferably approximately 100mg/l of ascorbic acid together with preferably 250mg/l of citric acid and most preferably approximately 150mg/l of citric acid is included in the media. After the initial stage of suspension culture, a concentration of 2mg/l to 20mg/l and most preferably approximately 10mg/l of ascorbic acid should be present in the maintenance medium to avoid browning.

Other anti-oxidants may be useful for example, activated charcoal may be incorporated in the initial solid medium before the callus is transferred to liquid media to initiate the suspension culture.

Alternatively both root and non-root explants are cultured together, as the presence of the root explant/callus substantially inhibits the occurrence of medium and cell browning.

An important factor influencing the formation of suspension cultures and their subsequent growth is the density of callus inoculum. The higher the density of inoculum the higher the frequency of cell aggregation. To obtain finely dispersed suspension culture, preferably the inoculum density is in the range 1 - 10g/l of medium. Most preferably the density is approximately 5g/l.

Most preferably the photoperiod for optimal growth of the suspension culture is 16 hours light/8 hour dark cycles.

Most preferably the carbohydrate source in the liquid culture media is sucrose. It may of course be other types of carbohydrate such as the above mentioned mono-and di-saccharides. For optimal embryo production, most preferably a sucrose and/or glucose concentration of 20 to 60 g/l and most preferably approximately 30g/l is used.

Preferably abscissic acid (ABA) is included in the liquid media. A concentration of between 0.1 and 20 mg/l is preferred and most preferably approximately 1 mg/l is preferable as this suppresses the growth of non-embryogenic cells whilst encouraging the growth of embryogenic cells, promoting the production of a substantially pure embryonic culture. This is particularly advantageous for efficient long term maintenance of the embryogenic culture as it avoids the need for the labour intensive removal of the non-embryogenic cells formed during each sub-culture to ensure efficient long-term regeneration. This use of ABA in culture media for the inhibition of growth of non-embryogenic cells could also be important in the culture of other types of plants.

For commercial large scale propagation of sugar cane embryos it is preferred, where the explant derived callus is initially grown on solid media for it to be transferred for further development in liquid culture. If the explant derived callus grown initially on solid media continues development on solid media the embryos do not develop synchronously and have to be individually handled, although they will eventually become established as sugar cane plantlets. Accordingly for commercial large scale propagation of sugar cane, it is preferable for the explant derived callus to be transferred from the solid media to liquid suspension culture for further development. However, importantly, it is alternatively possible to initiate immature globular embryo production directly from root-explants directly in liquid suspension culture avoiding the need for initial induction on solid media as required for example for at least shoot and leaf explant culture.

Step 2 of the method of the present invention accordingly most preferably involves the maturation of the immature globular embryos in liquid phase suspension culture to fully developed embryos by induction of somatic embryogenesis. Up until the present invention problems have prevented the successful production of mature sugar cane embryos in liquid culture.

The present invention provides a method of successfully achieving this using root, shoot and leaf explants as the initial plant tissue source. Most preferably root explants are used. Advantageously root-derived cultures in step 2 produce a mucilaginous material which renders the suspension viscous. This mucilaginous material has important properties which assist in prevention of browning. Furthermore the increased viscosity of the culture medium increases the degree of embryogenesis. Production of the mucilaginous material could be induced from sugar cane roots by submerging the roots in water at a relatively high temperature (such as 35°C) in sterile conditions. The mucilaginous material may then be retrieved and added to any plant tissue/cell culture suffering from "browning". The use of this material could replace the need for including expensive antioxidants in culture media normally required to avoid "browning".

As hereinbefore described, sugar cane immature embryos are effectively induced from explants in media comprising 2,4-D. Most preferably, for step 2 the immature embryos are subsequently transferred to a liquid medium free of 2,4-D auxin to encourage development of mature embryos. However, only root-derived suspension culture develops mature embryos in the liquid 2,4-D free media substantially without browning. Where leaf and shoot-derived suspension cultures, for example, are transferred to 2,4-D free media, browning develops which reduces the maturation of embryos. Accordingly, in such circumstances it is preferable to add activated charcoal or other suitable anti-oxidants at this stage to the media which assists in controlling browning of media and cells to allow and encourage mature embryos to develop. The addition of activated carbon/anti-oxidants is not a requirement for root-derived cultures but may be included in certain circumstances for example, when the root culture is older than approximately two months because beyond that time the root culture typically starts suffering from the problems of "browning". Most preferably, where activated charcoal is required it is added to the liquid media at a concentration of approximately 3 g/l.

Preferably the culture is allowed to develop over a period of time, preferably for between 50 and 60 days. During the maturation period preferably the media is changed frequently, for example weekly. The somatic embryogenesis of the immature globular embryos to bipolar and then to mature somatic torpedo embryos occurs in approximately 30-40 days depending on culture conditions, for example.

For somatic embryogenesis to be used for the large scale production of sugar cane plants, the mature embryos which are delivered to the field (preferably in the form of synthetic seeds) should produce a uniform growth of plants to facilitate agricultural operations such as weeding, irrigation and harvesting. In the case of sugar cane, plants must be at a certain age of maturity in order to achieve maximum sugar production. The sugar yield is reduced dramatically after flowering. It is therefore critical to have all the mature embryos for subsequent germination at the same stage of development so that the plants mature at the same time.

In order to avoid asynchronous maturation of the immature embryos, most preferably step 2 comprises a method of treating the embryos to overcome asynchronous development. Suitable treatments include storing the suspension culture in cold storage. Optimal conditions are for example, storage at 5°C (+ or - 1°C) for approximately 10 days. A further simple treatment which advantageously is easy to apply *in situ* without risk of contamination is heat treatment, for example at approximately 50°C for 45-60 mins. After this treatment non-embryogenic and embryogenic cells loose their viability whereas embryos are unaffected.

Following this heat treatment, or alternatively, preferably the suspension culture is sieved as described above in relation to the first step of the present invention in order to select for a culture having a higher percentage of somatic embryos. Optimally the suspension is sieved through a 63 µm mesh first sieve and collected on a 45 µm mesh second sieve.

Optimally, following sieving, the culture is agitated which substantially removes any remaining non-embryogenic cells. When the culture is within a stirred bioreactor this agitation is conveniently achieved by operating the stirrer at approximately 500 rpm for approximately 1 hour. It will be appreciated that the above methods of optimizing synchronous development of embryos in culture may be advantageously utilised for any type of plant culture where asynchronous development generally occurs for example in the culture of embryos for the production of trees, date palm and potato.

The production of sugar cane somatic embryos using bioreactors is highly desirable for the mass propagation of sugar cane plants by tissue and cell culture. Using a bioreactor, it is possible to control the culture conditions, automate the method, produce large volumes of suspension cultures and avoid the need to subculture the suspension cultures produced.

Bioreactors have many advantages over shake flasks for somatic embryogenesis. Apart from their large working volume, bioreactors provide a homogeneous culture because of the mixing produced by either mechanical stirring or by the aeration of the medium. Using bioreactors, it is possible to monitor pH, dissolved oxygen concentration and other environmental factors.

One of the most important factors is the dissolved oxygen concentration in the medium which is controlled by the rate of aeration and agitation in the bioreactor. Other factors of importance are the distribution and density of cells within the medium.

Any type of bioreactor is suitable for the method of the present invention such as air-lift bioreactors and mechanically stirred bioreactors. In an air-lift bioreactor air is used both to supply the aeration and to mix the culture. With the mechanically stirred bioreactor, it is possible to control the oxygenation of the medium independently of the level of mixing. This latter type of bioreactor is accordingly preferable as it is possible independently to vary the rotational speed of the mechanical stirrer. None of the non-embryogenic cells survive at rotor speeds in excess of 500 rpm so that variation of rotor speed is a valuable means of improving the synchrony of an embryogenic culture.

The percentage of somatic embryogenesis in the stirred-tank bioreactor is generally higher than that in the air-lift bioreactor. The stirred tank provides good mixing at all the stages whereas the flow rate in the air-lift bioreactor has to be increased continuously with increasing growth because many of the cells settle if the rate of airflow is low. Considerable evaporation and froth formation occurred with high airflow rates which causes many cells to be attached to the wall of the bioreactor.

According to a further preferred aspect of the present invention there is provided a method for the culture of sugar cane mature somatic embryos from immature embryos comprising the steps of:
(1) preparation of immature embryo liquid suspension culture from explant
(2) culturing the suspension in a bioreactor to form somatic embryos.

Most preferably the culture of step (1) and step (2) occurs in a bioreactor. However, step (1) could alternatively occur for example, in a shake flask and the immature embryo culture used as an innoculum for culture in a bioreactor in step (2).

Most preferably step (1) comprises direct culturing of root embryonic callus from a root explant in media comprising 2,4-D and ABA. Optimally the concentration of 2,4-D is in the range 0.1 to 5 mg/l and most preferably 3mg/l and the concentration of ABA is optimally 0.1 to 20 mg/l and most preferably 1mg/l. Preferably prior to step (2) the method comprises sieving the culture through a sieve to form a fine suspension in the above described manner. Preferably the sieve mesh size is 63 µm and the suspension is collected on 45 µm mesh size sieve, which substantially ensures that the fine suspension substantially comprises somatic cells. Subsequently the method preferably comprises an additional step of resuspending the culture of step 1 in fresh media prior to step 2. That additional step preferably comprises sub-culturing in a fresh media comprising 2,4-D and ABA for optimally approximately 1 month to form a highly embryonic culture. Most preferably the density of inoculation for resuspension in a bioreactor is approximately 5g/l. Any non-embryogenic cells remaining are most preferably removed by agitation of the culture at 500 rpm for 1 hour. This is most conveniently achieved in a stirred-tank type of bioreactor where the rotor speed of the stirrer can be adjusted accordingly.

Step (2) preferably comprises culturing the suspension in 2,4-D free medium comprising ABA, preferably in a stirred bioreactor. Most preferably the media in the bioreactor are regularly replaced until maturation occurs. This generally occurs in approximately 50 days. Preferably the media are replaced for example, every 10 days.

The mature embryos produced by the method of the present invention (in a bioreactor or otherwise) may subsequently be encapsulated to form "artificial seeds".

The production of sugar cane plantlets comprises the maturation of somatic embryos to plant seedlings by germination of the embryos. Most preferably this step comprises the culture of individual somatic embryos in liquid media. Preferably this culturing occurs on polythene foam. Polythene foam advantageously supports the seedlings allowing easy direct transfer of the seedling to the field. Preferably the somatic embryos are at a late torpedo stage. The embryos are preferably cultured in liquid 2,4-D free media. The media may advantageously include ABA at a concentration of 0.05-1mg/l and most preferably 0.1mg/l. The seedlings produced from embryos treated with ABA are found to be more vigorous and survive better in greenhouse conditions.

The somatic embryos produced by the method of the present invention may be encapsulated to form an artificial seed prior to their germination.

According to a further preferred aspect of the present invention there is provided a method for encapsulating a somatic embryo comprising the step of adding at least one encapsulating agent to a somatic embryo. The method is suitable for the encapsulation of many plant somatic embryos including, but not limited to, sugar cane, all types of trees, potatoes, date palm etc. to form so-called "artificial seeds" which are easy to handle, store and transport.

Most preferably the encapsulating agent comprises at least a whole cereal flour/water paste. Such a paste as an encapsulating agent is particularly advantageous since it provides nutrients for the enclosed embryo, allows aeration of the embryo, forms a crust to keep the embryo moist and easy germination for the embryo yet is inexpensive. Whole sorghum flour is a particularly preferred agent since it is particularly inexpensive. The flour used could be fermented or non-fermented. Fermented flour is most preferable since the capsules produced therefrom are substantially resistant to cracking. The flour to water mix is preferably 5 - 30 g flour per 100ml water and is most preferably approximately 10 g of flour to 100ml of water.

The method of encapsulation preferably comprises the steps of:
(1) forming a flour/water paste
(2) adding somatic embryos to the paste
(3) placing individual embryos encapsulated in paste into water to form a capsule around the or each embryo
(4) drying the capsule

The method of forming the flour/water paste preferably comprises mixing the flour and water together, heating the mixture to form a paste, autoclaving the paste, cooling the paste.

Prior to drying, the capsule could be dipped in a microbiocidal agent such as a fungicide and/or bacteriocide.

Before or after step (4) of the method of encapsulating the embryo the method could further comprise the steps of:
(a) adding sodium alginate solution to the capsule
(b) subsequently placing the capsule in calcium chloride solution.

Those additional steps produce beads of calcium alginate containing single embryos encapsulated in a flour paste.

Optimally, the concentration of sodium alginate solution used in the above mentioned step is approximately 3% which is transferred to, optimally, a 75mM solution of calcium chloride in step (ii). The beads of calcium alginate containing a single embryo may be allowed to germinate in liquid or solid media such as 2,4-D free media or directly in soil.

The method of this invention provides an artificial seed comprising a plant embryo encapsulated in a flour-based material. Preferably the flour-encapsulated embryo is enclosed within a calcium alginate bead. Most preferably the artificial seed is produced by the above described method.

Such artificial seeds comprising somatic embryos provide numerous advantages over sexual seeds. For example, a higher rate of germination occurs than with sexual seeds. Furthermore the artificial seeds are genetically stable and highly viable in contrast with the chromosomal variation and poor viability typically exhibited by sexual seeds. Furthermore, such artificial seeds provide numerous advantages over vegetive propogation of sugar cane which is costly in terms of sugar cane usage, labour and transport and also carries a high risk of disease contamination. The method of producing somatic embryos and subsequent encapsulation to form artificial seeds of the present invention provides a highly efficient method of sugar cane production as it provides a contamination free, low labour, time efficient method for the mass-production of viable genetically identical artificial seeds from a single sugar cane explant. The artificial seeds produced may be stored, transported and planted at minimal costs.

The plants produced by vegetative propagation of a mother plant are similar in morphology, sugar content, biochemistry etc as those produced by somatic embryogenesis in accordance with the present invention, and furthermore the plants produced by the method of the present invention are similar to the original mother plant in a wide variety of characteristics. Accordingly the method of the present invention provides a commercially important and viable new method for the large scale production of sugar cane.

The present invention will now be described by way of the following non-limiting examples.

### Examples

### Preparation of leaf explants

Sugar cane setts from variety Co527 (Kenana Sugar Company) were immersed in a water bath at 52°C for 2 days to expose any contamination and promote rooting and shooting.

The setts were subsequently grown on Levington Multipurpose Compost (Fisons) at 25°C with a 16 hour light photoperiod in a glasshouse from November to April and using natural daylight from May to October.

The growing plants were fertilized weekly with liquid fertilizer (Tomorite Rey TM, Fisons).

Leaf explants were excised at three months as 5mm segments of outer and inner leaves.

The leaf explants were sterilized by soaking in 95% ethanol for 20 mins.

### Preparation of shoot or root explants

Plantlets are obtained in vitro from a callus culture initiated from a leaf explant (variety Co527). A mother plant was selected from those plantlets and propagated on shoot multiplication medium. Root and shoot explants were obtained from such three month old micro-propagated plants. The root and shoot explant were transferred from regenerated plants in axenic cultures without sterilization.

### Preparation of Culture MS (Murashinge and Skoog, 1962) Medium

The basic medium was prepared from stock solutions and then supplemented with various combinations of plant growth regulators, vitamins and sugars, stock solutions of which were prepared separately and stored at -20°C in dark bottles. Myo-inosiltol, solidifying agent (agar) and carbohydrates were added to the media during preparation. The media were made up with double distilled water. Heat-stable plant growth regulators as well as other compounds were added before autoclaving, while heat labile compounds such as zeatin and ABA were added to the lukewarm media just before pouring into the sterile plates. All media were adjusted to pH 5.8 by using 1M MaOH or 1M HC1 prior to autoclaving. In the case of the solid medium, 0.9% (w/v) agar (Sigma, UK) was added before adjustment of the pH.

Murashige and Skoog (1962) medium with 30 g/l sucrose and 3mg/l 2,4-D was used for the growth and maintenance of the callus cultures, suspension cultures in shake flasks and bioreactors; it is referred to as (MS1). For Embryogenesis, Murashige and Skoog medium with 30 g/l sucrose was used and referred to as (MS2). Adjustments to the media may be made as required.

Periodic samples were taken aseptically and smeared onto plates of MYGP agar (malt and yeast extract, glucose and peptone), PDA (potato dextrose agar) and NA (nutrient agar) all supplied by Oxoid, Basingstoke, Hants. These plants were then incubated for 1 week at 25°C before being checked for microbial growth.

### Initiation of callus cultures

Callus cultures were initiated by culturing explants in 90 X 15 mm sterile plastic Petri dishes (Sterilin Ltd. UK) containing MS1 solidified with 0.9% agar. The Petri dishes were sealed with parafilm (American National Can Co.) to reduce loss of moisture by evaporation. After several weeks of incubation, when callus had grown, it was removed with sterile forceps and placed on fresh agar plates. These were then incubated for a further month. The remaining original explants, including any dark brown or contaminated tissues were discarded.

### Production of suspension cultures

Suspension cultures were initially produced by placing 0.5 - 1.0g (fresh weight) of callus material into sterile 250 ml Erlenmeyer shake flasks (Corning, Stone, Staffs.) containing 50 ml of MS1, After resealing with a double layer of sterilised aluminium foil squares of about 12 X 12 cm, the flasks were placed on rotary shakers at 100 rpm. Over several weeks, this gentle shaking action encouraged smaller fragments of cells and single cells to separate from the larger aggregates. The cultures were then sub-cultured, particular care being taken to transfer the smaller lumps and discard the larger aggregates. Aliquots of 10-15 ml of the finer cell aggregates were transferred to 50 ml fresh medium in 250 ml shake flasks every week.

### Direct initiation of Root Suspension Culture for small scale production

Roots either harvested from mature plants or from micropropagated plants were put directly into liquid medium which contains 0.5 -10mg/l 2,4-D (most preferably 3mg/l), and ABA 0.1 - 10 mg/l (most preferably 1mg/l). A fine suspension culture is formed.

### Production of mature embryos by somatic embryogenesis

For embryogenesis, cultures were transferred to MS medium free of 2,4-D. Maturation of embryos occurred in the same MS medium without growth regulators.

### Initiation of root suspension for industrial use (as inoculum for large bioreactors)

A well reared mother plant at the age of nine months is the source of the plant material. The plant was cut 10 cm above the ground a set with two nodes (about 20 cm long) was excised, the cut ends was waxed using a melted paraffin wax. The sett was sterilised in 20% sodium hypochlorite for 20 minutes and washed many times with distilled water, the waxed ends were removed. The buds were carefully removed to inhibit the growth of the shoots inside the bioreactor. The plant was placed inside a three litre bioreactor adjacent to the bioreactor wall and held to the wall by round silicon tube attached to the wall by means of non-toxic glue.

The bioreactor was filled with distilled water and the temperature was controlled by means of water-bath at 35°C with out running the stirrer. A massive root network grew. When the roots were approximately 1 inch long, the distilled water was siphoned out and the bioreactor was filled with MS1 medium. The temperature is subsequently reduced to 27°C. The bioreactor was stirred at 100 rpm. After 1 month a fine suspension free of browning was ready to be used as inoculum.

The fine suspension was sieved though 63 µm mesh sieve and collected on size 45 µm mesh sieve.

### Production of somatic embryos in 5 litres bioreactor for commercial use

From the above inoculum 25 g of cells was used to inoculate 5 litres stirred-tank bioreactor using MS2 medium. The stirrer was operated at 500 rpm which removed any non-embryogenic cells remaining. This procedure resulted in highly synchronized growth. At the end of the run 93% of the embryos were torpedo embryos at a density of approximately 1 million embryo per litre.

### Production of encapsulated somatic embryos

Sorghum flour (10g) was mixed with water (100ml) and the pH was adjusted to 5.8. The mixture was brought to boiling while continuously stirring to form a thick paste (it immediately solidified when a drop placed in water). At this stage the paste was autoclaved, at 15 psi, 121°C for 20 minutes. The paste was cooled to 40°C. For encapsulation, mature embryos were picked from the maturation medium after 3 weeks and then embryos were singly dropped in the paste. Using a pipette a rounded capsule comprising an embryo encapsulating the paste was dropped in cold water. At this stage the capsule could be dipped in fungicide and bactericide. Then the capsules were dried.

Sodium alginate (Sigma) was added at concentration 3.0,% (w/v) to MS2. The medium was autoclaved after adjusting the pH to 5.8. Flour-encapsulated embryos were first individually dipped in the sterile sodium alginate solution and then dropped in 75 mM calcium chloride solution in a beaker placed on a magnetic stirrer. The resultant beads, each containing a single somatic embryo were recovered by decanting the calcium chloride solution and washing with MS liquid medium.

### Germination of embryos to produce plantlets

After germination in MS (solid or liquid) medium, seedlings were transferred to perlite and then Levington compost (after 3 weeks) and grown on in a green house at 15-32°C and 87% relative humidity. Long day conditions (16 hours light) were maintained under mercury vapour lamps. After 3 weeks, the plantlets were transferred to compost).

### Viability test

Viability of cells and embryos in culture was assessed using the fluorescein diacetate (FDA) test (Widholm, 1972). A solution of FDA in acetone (5 mg/ml) was diluted in water to give a final concentration of 0.01% before use. Equal volumes of this solution and the cell suspension were mixed and left at room temperature for at least 3 minutes. One drop of the mixture was pipetted onto a glass microscope slide, covered with a glass coverslip and viewed under an Olympus BH2 U.V. microscope fitted with a model BH-RFL-W reflected light fluorescence attachment, 100 watt mercury vapour lamp, EY.455 exciter filter, B(DM.500+ 0.515) dichromatic mirror and a 530nm barrier filter. After several minutes to allow the fluorescent to develop, the numbers of yellow-green fluorescing cells (viable) and the total number of cells including non-fluoresencing (non-viable) cells were counted in 10 random fields.).

It will be appreciated by those skilled in the art that the present invention provides a time and labour efficient, yet reliable method suitable for the large scale production of sugar cane mature embryos. Advantageously the method is suitable for production to occur in a bioreactor where the method may be fully automated.

It is to be understood that the scope of the invention should not be limited to the above described examples, as those examples are for illustration by way of example only.

## Claims

1. A method comprising production of sugar cane somatic embryos from sugar cane explants further comprising the steps of:
(1) culturing immature embryos from explants;
(2) culturing mature sugar cane somatic embryos from those immature embryos,
and **characterised in that** at least step (2) occurs in liquid suspension culture.

2. A method according to claim 1 **characterised in that** both the growth of embryogenic tissue in step (1) and the subsequent development of the embryos to maturity in step (2) occur in liquid media.

3. A method according to any one of claims 1 or 2 **characterised in that** the sugar cane plants are derived from characteristic organised meristems or mestematic cells of sugar cane plants.

4. A method according to any one of claims 1 to 3 **characterised in that** the explant material comprises root derived or mature embryos.

5. A method according to claim 4 **characterised in that** the explant material comprises mature embryos derived by stressing sugar cane plant material.

6. A method according to claim 5 **characterised in that** stressing is achieved by cooling the plant material.

7. A method according to claim 6 **characterised in that** the plant material is cooled to 5-15°C for 1-3 months.

8. A method according to any one of claims 5 to 7 **characterised in that** stressing is achieved by treatment with alcohol.

9. A method according to any one of claims 1 to 8 **characterised in that** the method of culturing immature embryos from explants (step 1) comprises the steps of:
a) culturing explant material on culture medium to produce somatic cells
b) selecting and multiplying somatic cells derived from the explant material
c) culturing the somatic cells for a period of time as culture medium to induce immature sugar cane embryos from the somatic cells.

10. A method according to any one of claims 1 to 9 **characterised in that** the immature embryos are initiated and maintained as embryogenic callus on solid media or as an embryogenic cell suspension culture in liquid media or in a combination of both solid and liquid media.

11. A method according to any one of claims 1 to 10 **characterised in that** plant growth regulators such as auxins, cytokinins, gibberellins, abscissic acid, anti-auxins, kinetin, zeatin and/or activated charcoal are included either alone or in combination in the media formulation.

12. A method according to any one of claims 1 to 11 **characterised in that** it is performed in a medium containing sucrose as a major carbohydrate constituent.

13. A method according to claim 12 **characterised in that** the concentration of sucrose used is in the range of 10-60 g/l.

14. A method according to any one of claims 1 to 13 **characterised in that** explant material is derived from sugar cane plant tissue having exposed cut ends coated with wax.

15. A method according to any one of claims 1 to 14 **characterised in that** a selection of substantially only somatic cells is achieved by applying at least one of the following: sieving culture material, heat treatment or stirring.

16. A method according to claim 15 using a first sieve of 100-50 µm mesh size and subsequently a second sieve having a mesh size of 50-38 µm.

17. A method according to any one of claims 1 to 16 **characterised by** the use of light and dark cycles during production of the immature embryos.

18. A method according to any one of claims 1 or 2 and 7 to 17 **characterised in that** the culturing of immature embryos involves growth of sub-cultures from the initial culture using fresh culture media for the sub-culture.

19. A method according to any one of claims 1 to 18 **characterised by** the initial culture of explant involving the initiation of embryogenic callus from explants on solid media followed by the sub-culturing of embryogenic callus from the explant in liquid media to establish and maintain embryogenic cell suspension cultures.

20. A method according to any one of claims 1 to 19 **characterised in that** where leaf explant derived cultures are used, ascorbic acid is included as a constituent of the callus initiation solid medium and the liquid embryogenic suspension medium.

21. A method according to claim 20 **characterised in that** the initiation of suspension culture from callus includes approximately 50mg/l to 200mg/l of ascorbic acid.

22. A method according to claim 20 or 21 **characterised in that** the initiation of suspension culture from callus includes ascorbic acid and citric acid.

23. A method according to any one of claims 1 to 22 **characterised in that** abscissic acid is included in a liquid culture medium.

24. A method according to any one of claims 1 to 23 **characterised in that** the culture of sugar cane mature somatic embryos from immature embryos comprises the steps of:
(1) preparation of immature embryo liquid suspension culture from explant;
(2) culturing the suspension in a bioreactor to form somatic embryos.

25. A method according to claim 24 **characterised in that** the culture of step (1) as well as step (2) occurs in a bioreactor.

26. A method according to any preceding claim **characterised in that** produced mature embryos are encapsulated prior to their germination.

27. A method according to claim 26 **characterised in that** the encapsulating agent comprises a cereal flour/water paste.

28. A method according to claim 27 **characterised in that** the flour is sorghum flour.

29. A method according to any one of claims 1 to 28 **characterised in that** root derived cultures used in step (2) produce a mucilaginous material, production of which is induced by submerging the roots in water at a temperature of approximately 35°C in sterile conditions.

30. A method according to claim 29 **characterised in that** for step (2) root derived immature embryos are subsequently transferred to a liquid medium substantially free of 2,4-D auxin.

31. A method according to any one of claims 1 to 30 **characterised in that** for step (2) leaf and shoot derived suspension culture transferred to a 2,4-D auxin free media, activated charcoal or other antioxidant is included in the medium.

32. A method according to claim 26 **characterised in that** encapsulation comprises the steps of:
(1) forming a flour/water mix
(2) adding somatic embryos to the paste
(3) placing individual embryos encapsulated in paste into water to form a capsule around the embryo(s)
(4) drying the capsule.

33. A method according to claim 32 **characterised in that** prior to drying (step (4)) the capsule is dipped in a microbiocidal agent such as a fungicide and/or bacteriocide.

34. A method according to claim 32 **characterised in that** prior to drying (step (4)) the encapsulating of the embryo further comprises the steps of:
a) adding sodium alginate solution to the capsule
b) subsequently placing the capsule in calcium chloride solution.

35. A method according to any one of claims 1 to 34 **characterised in that** sugar cane plantlets are produced by maturation of somatic embryos to plant seedlings by germination of embryos, whereby individual somatic embryos are cultured in liquid media carried on polythene foam.

36. A method according to claim 35 **characterised in that** the somatic embryos are at a late torpedo stage and are cultured in 2,4-D auxin free liquid media and this liquid media includes abscissic acid (ABA).

37. The method any one of claims 1 to 36 when applied to plant material derived from plant sources other than sugar cane consisting of trees, date palm and potato.

## Patentansprüche

1. Verfahren umfassend die Herstellung von somatischen Embryonen des Zuckerrohrs aus Zuckerrohrexplantaten, des Weiteren enthaltend die Schritte
1. Kultivieren unreifer Embryonen aus Explantaten,
2. Kultivieren reifer somatischer Embryonen des Zuckerrohrs aus diesen unreifen Embryonen,
und **dadurch gekennzeichnet, dass** zumindest Schritt 2 in einer flüssigen Suspensionskultur durchgeführt wird.

2. Verfahren nach Anspruch 1, **dadurch gekennzeichnet, dass** sowohl das Wachstum des embryogenen Gewebes in Schritt 1 als auch die nachfolgende Entwicklung der Embryonen zur Reife im flüssigen Medium stattfinden.

3. Verfahren nach einem der Ansprüche 1 oder 2, **dadurch gekennzeichnet, dass** die Zuckerrohrpflanzen von charakteristisch aufgebauten Meristemen oder von meristematischen Zellen von Zuckerrohrpflanzen abgeleitet werden.

4. Verfahren nach einem der Ansprüche 1 bis 3, **dadurch gekennzeichnet, dass** das explantierte Material Wurzel-abgeleitete oder reife Embryonen enthält.

5. Verfahren nach Anspruch 4, **dadurch gekennzeichnet, dass** das explantierte Material reife Embryonen enthält, welche mittels Stressbelastung des Pflanzenmaterials des Zuckerrohrs gewonnen werden.

6. Verfahren nach Anspruch 5, **dadurch gekennzeichnet, dass** die Stressbelastung durch Kühlen des Pflanzenmaterials erreicht wird.

7. Verfahren nach Anspruch 6, **dadurch gekennzeichnet, dass** das Pflanzenmaterial für ein bis drei Monate auf eine Temperatur von 5 bis 15°C gekühlt wird.

8. Verfahren nach einem der Ansprüche 5 bis 7, **dadurch gekennzeichnet, dass** die Stressbelastung durch eine Behandlung mit Alkohol erzielt wird.

9. Verfahren nach einem der Ansprüche 1 bis 8, **dadurch gekennzeichnet, dass** das Verfahren zur Kultivierung unreifer Embryonen aus Explantaten (Schritt 1) die Schritte umfasst:
a) Kultivieren explantierten Materials in Kulturmedium um somatische Zellen zu produzieren,
b) Auswählen und Vermehren somatischer Zellen, welche aus dem explantierten Material gewonnen werden,
c) Kultivieren der somatischen Zellen als Kulturmedium für einen Zeitraum um unreife Zuckerrohrembryos aus den somatischen Zellen hervorzurufen.

10. Verfahren nach einem der Ansprüche 1 bis 9, **dadurch gekennzeichnet, dass** die unreifen Embryonen als embryogener Callus auf festem Medium oder als eine embryogene Zellsuspensionskultur in flüssigem Medium oder in einer Kombination aus sowohl festem als auch flüssigem Medium initiiert und erhalten werden.

11. Ein Verfahren nach einem der Ansprüche 1 bis 10, **dadurch gekennzeichnet, dass** Pflanzenwachstumsregulatoren wie Auxine, Cytokinine, Gibberelline, Abscissinsäure, Anti-Auxine, Kinetin, Zeatin und/oder Aktivkohle entweder einzeln oder in Kombination in der Zusammensetzung des Mediums enthalten sind.

12. Verfahren nach einem der Ansprüche 1 bis 11, **dadurch gekennzeichnet, dass** es in einem Medium enthaltend Saccharose als hauptsächlichen Kohlenhydratbestandteil durchgeführt wird.

13. Verfahren nach Anspruch 12, **dadurch gekennzeichnet, dass** die Konzentration der verwendeten Saccharose im Bereich von 10 bis 60 g/l liegt.

14. Verfahren nach einem der Ansprüche 1 bis 13, **dadurch gekennzeichnet, dass** das explantierte Material von Rohrzuckerpflanzengewebe gewonnen wird ist, bei welchem die freien abgeschnittenen Enden mit Wachs überzogen sind.

15. Verfahren nach einem der Ansprüche 1 bis 14, **dadurch gekennzeichnet, dass** eine Auswahl von im Wesentlichen nur somatischen Zellen dadurch erreicht wird, dass mindestens eines der Folgenden angewendet wird: Sieben des Kulturmaterials, Hitzebehandlung oder Rühren.

16. Verfahren nach Anspruch 15, wobei ein erstes Sieb mit einer MaschengrRße von 100 bis 50 µm und anschließend ein zweites Sieb mit einer MaschengrRße von 50 bis 38 µm verwendet wird.

17. Verfahren nach einem der Ansprüche 1 bis 16, **dadurch gekennzeichnet, dass** Hell- und Dunkel-Zyklen während der Produktion der unreifen Embryonen eingesetzt werden.

18. Verfahren nach einem der Ansprüche 1 oder 2 sowie 7 bis 17, **dadurch gekennzeichnet, dass** das Kultivieren unreifer Embryonen Wachstum von Sub-Kulturen aus der Erstkultur umfasst, wobei frisches Kulturmedium für die Sub-Kultur eingesetzt wird.

19. Verfahren nach einem der Ansprüche 1 bis 18, **dadurch gekennzeichnet, dass** die Erstkultur des Explantats das Initiieren von embryogenem Callus aus Explantaten auf festem Medium umfasst, welches gefolgt wird vom Sub-Kultivieren von embryogenem Callus aus dem Explantat in flüssigem Medium, um eine embryogene Zellsuspensionskultur einzurichten und zu erhalten.

20. Verfahren nach einem der Ansprüche 1 bis 19, **dadurch gekennzeichnet, dass** bei der Verwendung von aus Pflanzenexplantaten gewonnenen Kulturen das feste Medium zur Callusinitiierung und das flüssige embryogene Suspensionsmedium Ascorbinsäure als Bestandteil enthält.

21. Verfahren nach Anspruch 20, **dadurch gekennzeichnet, dass** zur Initiierung der Suspensionskultur aus Callus ungefähr 50 mg/l bis 200 mg/l Ascorbinsäure eingesetzt wird.

22. Verfahren nach einem der Ansprüche 20 oder 21, **dadurch gekennzeichnet, dass** zur Initiierung der Suspensionskultur Ascorbinsäure und Zitronensäure eingesetzt wird.

23. Verfahren nach einem der Ansprüche 1 bis 22, **dadurch gekennzeichnet, dass** Abscisinsäure in einem flüssigen Kulturmedium enthalten ist.

24. Verfahren nach einem der Ansprüche 1 bis 23, **dadurch gekennzeichnet, dass** das die Kultur reifer somatischer Embryonen des Zuckerrohrs aus unreifen Embryonen die Schritte aufweist:
1. Herstellung flüssiger Suspensionskultur unreifer Embryonen aus Explantaten
2. Kultivieren der Suspension in einem Bioreaktor zur Gewinnung von somatischen Embryonen.

25. Verfahren nach Anspruch 24, **dadurch gekennzeichnet, dass** sowohl Schritt 1 als auch Schritt 2 in einem Bioreaktor stattfinden.

26. Verfahren nach einem der vorhergehenden Ansprüche, **dadurch gekennzeichnet, dass** die produzierten reifen Embryonen vor ihrer Keimung eingekapselt werden.

27. Verfahren nach Anspruch 26, **dadurch gekennzeichnet, dass** das Einkapselungsmittel eine Getreidemehl/Wasser-Paste enthält.

28. Verfahren nach Anspruch 27, **dadurch gekennzeichnet, dass** das Mehl Sorghummehl ist.

29. Verfahren nach einem der Ansprüche 1 bis 28, **dadurch gekennzeichnet, dass** die in Schritt 2 verwendeten aus Wurzel gewonnenen Kulturen ein schleimiges Material produzieren, dessen Produktion durch Eintauchen der Wurzeln in Wasser bei einer Temperatur von ungefähr 35°C unter sterilen Bedingungen eingeleitet wird.

30. Verfahren nach Anspruch 29, **dadurch gekennzeichnet, dass** für Schritt 2 aus Wurzel gewonnene unreife Embryonen anschließend in ein flüssiges Medium überführt werden, das weitgehend frei von 2,4-D Auxin ist.

31. Verfahren nach einem der Ansprüche 1 bis 30, **dadurch gekennzeichnet, dass** für Schritt 2 die aus Blatt und Stengel gewonnene und in ein 2,4-D Auxinfreies Medium überführte Suspensionskultur Aktivkohle oder ein anderes Antioxidans im Medium enthält.

32. Verfahren nach Anspruch 26, **dadurch gekennzeichnet, dass** die Einkapselung die folgenden Schritte umfasst:
1. Herstellung einer Mehl/Wasser-Paste,
2. Zusetzen somatischer Embryonen zu der Paste,
3. Einsetzen einzelner in Paste eingekapselter Embryonen in Wasser, um eine Kapsel um den Embryo (die Embryonen) zu bilden,
4. Trocknen der Kapsel.

33. Verfahren nach Anspruch 32, **dadurch gekennzeichnet, dass** vor dem Trocknen nach Schritt 4 die Kapsel in ein mikrobizides Mittel wie ein Fungizid und/oder ein Bakterizid eingetaucht wird.

34. Verfahren nach Anspruch 3, **dadurch gekennzeichnet, dass** vor dem Trocknen gemäss Schritt 4 das Einkapseln der Embryonen weiter folgende Schritte umfasst:
a) Hinzufügen von NatriumalginatlRsung zu den Kapseln,
b) anschließendes Einsetzen der Kapsel in CalciumchloridlRsung.

35. Verfahren nach einem der Ansprüche 1 bis 34, **dadurch gekennzeichnet, dass** Zuckerrohrpflänzchen hergestellt werden durch Reifung von somatischen Embryonen zu Pflanzensämlingen aufgrund von Keimung von Embryonen, wobei einzelne somatische Embryonen in flüssigem Medium auf Polyethylenschaum kultiviert werden.

36. Verfahren nach Anspruch 35, **dadurch gekennzeichnet, dass** sich die somatischen Embryonen in einer späten Torpedostufe und in einem 2,4-D Auxin-freiem flüssigen Medium befinden, und das dieses Flüssigmedium Abscisinsäure (ABA) enthält.

37. Verfahren nach einem der Ansprüche 1 bis 36, **dadurch gekennzeichnet, dass** sie auf Pflanzenmaterial angewendet werden, welches von anderen pflanzlichen Quellen als Zuckerrohr, die Bäume, Dattelpalmen und Kartoffeln umfassen, abgeleitet wird.

## Revendications

1. Procédé comprenant la production d'embryons somatiques de canne à sucre à partir d'explants de canne à sucre comprenant en outre les étapes :
(1) de culture d'embryons immatures à partir d'explants
(2) de culture d'embryons somatiques matures de canne à sucre à partir de ces embryons immatures,
et **caractérisé en ce qu'**au moins l'étape (2) est réalisée dans une culture en suspension dans un liquide.

2. Procédé selon la revendication 1, **caractérisé en ce que** la croissance du tissu embryogénique dans l'étape (1) et le développement consécutif des embryons vers la maturité dans l'étape (2) sont réalisés dans un milieu liquide.

3. Procédé selon l'une quelconque des revendications 1 ou 2, **caractérisé en ce que** les plants de canne à sucre sont dérivés de méristèmes organisés caractéristiques ou de cellules méristématiques de plantes de canne à sucre.

4. Procédé selon l'une quelconque des revendications 1 à 3, **caractérisé en ce que** le matériau d'explant comprend des embryons dérivés de racines ou matures.

5. Procédé selon la revendication 4, **caractérisé en ce que** le matériau d'explant comprend des embryons matures dérivés en forçant le matériau végétal de canne à sucre.

6. Procédé selon la revendication 5, **caractérisé en ce que** le forçage est réalisé en refroidissant le matériau végétal.

7. Procédé selon la revendication 6, **caractérisé en ce que** le matériau végétal est refroidi entre 5 et 15°C pendant 1 à 3 mois.

8. Procédé selon l'une quelconque des revendications 5 à 7, **caractérisé en ce que** le forçage est réalisé par traitement avec de l'alcool.

9. Procédé selon l'une quelconque des revendications 1 à 8, **caractérisé en ce que** le procédé de culture d'embryons immatures d'explants (étape 1) comprend les étapes :
a) de culture de matériau d'explant sur un milieu de culture pour produire des cellules somatiques,
b) de sélection et de multiplication de cellules somatiques dérivées du matériau d'explant
c) de culture des cellules somatiques pendant un certain laps de temps comme milieu de culture pour induire des embryons immatures de canne à sucre à partir des cellules somatiques.

10. Procédé selon l'une quelconque des revendications 1 à 9, **caractérisé en ce que** les embryons immatures sont initiés et maintenus comme cals embryogéniques sur un milieu solide ou comme une culture en suspension de cellules embryogéniques en milieu liquide ou dans une combinaison de milieu solide et liquide.

11. Procédé selon l'une quelconque des revendications 1 à 10, **caractérisé en ce que** des régulateurs de croissance de végétaux tels que des auxines, des cytokines, des gibbérellines, l'acide abscissique, des anti-auxines, la kinétine, la zéatine et/ou le charbon actif sont inclus, seuls ou en combinaison dans la formulation du milieu.

12. Procédé selon l'une quelconque des revendications 1 à 11, **caractérisé en ce qu'**il est réalisé dans un milieu contenant du sucre comme constituant hydrate de carbone majeur.

13. Procédé selon la revendication 12, **caractérisé en ce que** la concentration utilisée en sucre est dans la plage de 10 à 60 g/l.

14. Procédé selon l'une quelconque des revendications 1 à 13, **caractérisé en ce que** le matériau d'explant est dérivé de tissu végétal de canne à sucre présentant des extrémités coupées exposées revêtues de cire.

15. Procédé selon l'une quelconque des revendications 1 à 14, **caractérisé en ce qu'**on réalise une sélection substantiellement uniquement des cellules somatiques en appliquant au moins l'une parmi les techniques suivantes : criblage du matériau de culture, traitement thermique ou agitation.

16. Procédé selon la revendication 15, utilisant un premier tamis avec des mailles de 100 à 50 µm et ensuite un deuxième tamis présentant des mailles de 50 à 38 µm.

17. Procédé selon l'une quelconque des revendications 1 à 16, **caractérisé par** l'utilisation de cycles de lumière et d'obscurité pendant la production d'embryons immatures.

18. Procédé selon l'une quelconque des revendications 1 ou 2 et 7 à 17, **caractérisé en ce que** la culture des embryons immatures comprend la croissance de sous-cultures de la culture initiale en utilisant un milieu de culture frais pour la sous-culture.

19. Procédé selon l'une quelconque des revendications 1 à 18, **caractérisé par** la culture initiale d'explants comprenant l'initiation de cals embryogéniques d'explants sur un milieu solide suivie de la sous-culture de cals embryogéniques de l'explant dans un milieu liquide pour réaliser et maintenir des cultures en suspension de cellules embryogéniques.

20. Procédé selon l'une quelconque des revendications 1 à 19, **caractérisé en ce que**, lorsqu'on utilise des cultures dérivées d'explants de feuilles, de l'acide ascorbique est compris comme constituant du milieu solide d'initiation des cals et du milieu en suspension embryogénique liquide.

21. Procédé selon la revendication 20, **caractérisé en ce que** l'initiation de la culture en suspension à partir de cals comprend environ 50 mg/l à 200 mg/l d'acide ascorbique.

22. Procédé selon la revendication 20 ou 21, **caractérisé en ce que** l'initiation de la culture en suspension à partir de cals comprend de l'acide ascorbique et de l'acide citrique.

23. Procédé selon l'une quelconque des revendications 1 à 22, **caractérisé en ce que** l'acide abscissique est compris dans un milieu de culture liquide.

24. Procédé selon l'une quelconque des revendications 1 à 23, **caractérisé en ce que** la culture des embryons somatiques matures de canne à sucre à partir d'embryons immatures comprend les étapes :
(1) de préparation d'une culture en suspension liquide d'embryons immatures à partir d'explants,
(2) de culture de la suspension dans un bioréacteur pour former des embryons somatiques.

25. Procédé selon la revendication 24, **caractérisé en ce que** la culture de l'étape (1) ainsi que de l'étape (2) est réalisée dans un bioréacteur.

26. Procédé selon l'une quelconque des revendications précédentes, **caractérisé en ce que** les embryons matures produits sont encapsulés avant leur germination.

27. Procédé selon la revendication 26, **caractérisé en ce que** l'agent d'encapsulation comprend une pâte farine de céréale/eau.

28. Procédé selon la revendication 27, **caractérisé en ce que** la farine est la farine de sorgho.

29. Procédé selon l'une quelconque des revendications 1 à 28, **caractérisé en ce que** les cultures dérivées de racine utilisées dans l'étape (2) produisent un matériau mucilagineux, dont la production est induite en immergeant les racines dans l'eau à une température d'environ 35°C dans des conditions stériles.

30. Procédé selon la revendication 29, **caractérisé en ce que** pour l'étape (2), les embryons immatures dérivés de racine sont consécutivement transférés dans un milieu liquide substantiellement exempt de 2,4-D-auxine.

31. Procédé selon l'une quelconque des revendications 1 à 30, **caractérisé en ce que** pour l'étape (2), une culture en suspension dérivée de feuilles et de pousses transférée dans un milieu exempt de 2,4-D-auxine, du carbone actif ou un autre antioxydant est compris dans le milieu.

32. Procédé selon la revendication 26, **caractérisé en ce que** l'encapsulation comprend les étapes :
(1) de formation de pâte farine/eau
(2) d'addition d'embryons somatiques à la pâte
(3) de placement des différents embryons encapsulés dans la pâte dans de l'eau pour former une capsule autour du ou des embryons
(4) de séchage de la capsule.

33. Procédé selon la revendication 32, **caractérisé en ce qu'**avant le séchage (étape (4)), la capsule est plongée dans un agent micriobiocide tel qu'un fongicide et/ou un bactériocide.

34. Procédé selon la revendication 32, **caractérisé en ce qu'**avant le séchage (étape (4)), l'encapsulation de l'embryon comprend en outre les étapes
a) d'addition d'une solution d'alginate de sodium à la capsule.
b) de placement consécutif de la capsule dans une solution de chlorure de clacium.

35. Procédé selon l'une quelconque des revendications 1 à 34, **caractérisé en ce que** les plantules de canne à sucre sont produites par maturation d'embryons somatiques en germes végétaux par germination d'embryons, des embryons somatiques individuels étant cultivés dans un milieu liquide supporté sur une mousse de polyéthylène.

36. Procédé selon la revendication 35, **caractérisé en ce que** les embryons somatiques sont à un stade de torpille tardive et sont cultivés dans un milieu liquide exempt de 2,4-D-auxine et ce milieu contient de l'acide abscissique (ABA).

37. Procédé selon l'une quelconque des revendications 1 à 36, lorsqu'il est appliqué à un matériau végétal dérivé de sources végétales autres que la canne à sucre constituées d'arbres, de palmiers à dattes et de pommes de terre.
